# EUROPEAN PATENT APPLICATION

(11) **EP 1 905 845 A2**
(43) Date of publication of application: **02.04.2008**
(21) Application number: 07018522.8
(22) Date of filing: 20.09.2007
(51) Int. Cl.: C12Q 1/68, A61K 31/7088, C12N 15/11

(54) **Method for detecting multiple myeloma and method for inhibiting the same**

(30) Priority: 21.09.2006 JP 2006255155
(71) Applicant: FUJIFILM Corporation, Minato-ku Tokyo (JP); Tokyo Medical and Dental University, Tokyo 113-8510 (JP)
(72) Inventor: Zhao, Chen, Bunkyo-ku Tokyo 113-8510 (JP); Inoue, Jun, Bunkyo-ku Tokyo 113-8510 (JP); Imoto, Issei, Bunkyo-ku Tokyo 113-8510 (JP); Inazawa, Johji, Bunkyo-ku Tokyo 113-8510 (JP)
(74) Representative: HOFFMANN EITLE

(57) **Abstract**

It is an object of the present invention to identify a gene that relates to exhibition of behavior peculiar to cancer such as multiple myeloma to provide a method for detecting cancer and a cell-growth inhibitor. The present invention provides a method for detecting cancer which comprises detecting and/or typing tumorigenesis of a specimen by employing gene amplification in the chromosome 11 q23 region of the specimen as an indicator.

SEQUENCE LISTING
<110> FUJIFILM Corporation
<120> A method for detecting and suppressing multiple myeloma
<130> A61517A
<160> 4
<170> PatentIn version 3.1
<210> 1
<211> 3314
<212> DNA
<213> Homo sapiens
<400> 1
gaattccggc aggcgcccat ggcgggctga gtcctgccca tgccctggtg gcctggaagc 60
ctgcatgggc gccgtgcaaa gatcaacgtt ttccagagga gcttttgggc tcatcactgg 120
cctggggagg gcttgacacg gggttcccac agcttgcccc acaccacctt cacacccaca 180
cacagcaggt ccccgcgggg ccggtgcagg ctgtggctgc ccgcgctgcg ctgttgctct 240
gtcccggctg gctggctccg agtgtggggc gctctgggcc ggggccgctg gggcgcgcac 300
agtggggtga caggcggcct ggctgcagaa acgttgcacc ggtgcctgag gtgggaggat 360
gtgatgacgc ggcccacgca gcgggaaccc aggcctttaa aaagcccagg aaacagcctc 420
agctcaagcg gtggctccac tggaggaaaa cacaccccgg tctcacatta aagaagccaa 480
actgtcggct tcaaagagaa aaggcaacat cctgtcacag gccatgctct ggcaaaaacc 540
cacagctccg gagcaagccc cagccccggc ccggccatac cagggcgtcc gtgtgaagga 600
gccagtgaag gaactgctga ggaggaagcg aggccacgcc agcagtgggg cagcacctgc 660
acctacggcg gtggtgctgc cccatcagcc cctggcgacc tacaccacag tgggtccttc 720
ctgcctggac atggaaggtt ctgtgtctgc agtgacagag gaggctgccc tgtgtgccgg 780
ctggctctcc cagcccaccc cggccaccct gcagcccctg gccccatgga caccttacac 840
cgagtatgtg ccccatgaag ctgtcagctg cccctactca gctgacatgt atgtgcagcc 900
cgtgtgcccc agctacacgg tggtggggcc ctcctcagtg ttggcctatg cctctccgcc 960
actcatcacc aatgtcacga caagaagctc cgccacgccc gcagtggggc ccccgctgga 1020
gggcccagag caccaggcac ccctcaccta tttcccgtgg cctcagcccc tttccacact 1080
acccacctcc accctgcagt accggcctcc ggccccagcc ctacctgggc cccagtttgt 1140

ccagctcccc atctctatcc cagagccagt ccttcaggac atggaagacc ccagaagagc 1200
cgccagctcg ttgaccatcg acaagctgct tttggaggaa gaggatagcg acgcctatgc 1260
gcttaaccac actctctctg tggaaggctt ttaggcgtgg ctcccacctg agtcctgttc 1320
cctgaaactg ggattttaaa atgagcctgg aattgagccc caggttcatg cttgtttgga 1380
gtagtcattt catgactact ctttctacgc acagctagaa ttgtagacct gtaaaccttc 1440
cttcccttct tccttcccct ccctccctca cttcctccct ctcccccatc cttccattct 1500
tcccctccct cccttcttcc tttccaaccc cttccttcct tttcctccct cccttccttc 1560
cctccttccc ttctcccttt ccaacccctt ccttcctttt cctccctccc ttccttccct 1620
ccttcccttc tccctttcct tctttacctc cctccctccc tcgcttcttc tctctttctt 1680
acttcttttt tcaattctgt tccattttgg gaggtaatta tagggatttt agcaataaca 1740
ttttatgtca aatgttgcca agtctgtggt ccatgggctt tcatttctgt cacatttcat 1800
ttcttggaaa aggcctcctt cctccagtgc ctgctgaacc atcttagggt cactcacacc 1860
ctctgtaatt ttaagatgta tgtggtggcc ggcgggaaga ccagccccga cagcacctcc 1920
tgagaaagtc agccaagggc ctaccctgat gccagagtcc ttgagctgtc agttcccaca 1980
gttgctcctt tgtttgctct tctcagcctc ggccagattt acagtccagg cagcaaaatc 2040
tcaaggcctg gggctcagag tagtaagggg tgggaagtgg gtggcaggga gaaaagaaca 2100
tcagggtggg tggggacagg ccagtgacga agagagggac agaggaggga tgggaacagg 2160
ctgtgcatct tagttggaga gaggggtgtg ggaggaagct tgagtttgat gcagggagga 2220
ggaaggctga ggaatgactt ggctccagat tacttggtta ttaagaagaa caataaacta 2280
aaggaaagca ttgcttgaag agatggtttt gctgctctcc ttgaggatac gtgcaaggga 2340
agttgggctg ttgtaaacag ggtgaagggt gtgtttggtc ggccatttct ctctcacctc 2400
taggccctct gctggtgctg tggaggccaa gaccccatta agcctaaagg tgatgggtcc 2460
tcgcctaggc ttagtgctac catgtgggtt ttgtttcttt ccttccttcc ttccttcctt 2520
ccttccttcc ttccttcctt ccttccttcc ttccttcctt ccttctttcc ttccttcctt 2580
ccttccttcc ttcctctctc tttctttctt ttttttggtt ttttttggga cggagtctca 2640
ctccattgcc caggctggag tacaatggtg cgatcttggc tcactgcacc tctgtctccc 2700
aagttcaagt gattctcctg cctgagcctc ccgagtagct gggattacag gtgcatgcca 2760
ccatgcctgg ctaatttttg tatttttagt agagacgggg tttcaccatg ttggccaggc 2820
tggtctcgaa ctcctgacct tgtgatccgc gcgcctcagc ctctcagagt gctgggatca 2880
caggcgtgag ccaccgcacc cagcctttta ccatgtgggt ttctttagtg tcttaaaagc 2940

gtccataagc caccattctg tggaaccaag gccccctcca cgcaaacacc ctccctcctg 3000
gggacctctg gagcctcagc cagaagtacc attaggttta attttaattt gttttgctgg 3060
agaaacatca ggtttgtagg agactgagtt gttagcaggt gtgcttagct cttgatagtg 3120
aacgtgtacc ttgggaactg gctcacccac ctgctaatag caccatcgtc actattaagc 3180
agacatttca gttggtagaa tccatgtaga agtcatggac ttttctggga aatgactttt 3240
ctgggaaatg acagtttctt tgacatattt tctttgccca ctttaaataa aaactctgga 3300
gaaaaaaaaa aaaa 3314
<210> 2
<211> 256
<212> PRT
<213> Homo sapiens
<400> 2
Met Leu Trp Gln Lys Pro Thr Ala Pro Glu Gln Ala Pro Ala Pro Ala
1               5                   10                  15
Arg Pro Tyr Gln Gly Val Arg Val Lys Glu Pro Val Lys Glu Leu Leu
            20                  25                  30
Arg Arg Lys Arg Gly His Ala Ser Ser Gly Ala Ala Pro Ala Pro Thr
        35                  40                  45
Ala Val Val Leu Pro His Gln Pro Leu Ala Thr Tyr Thr Thr Val Gly
    50                  55                  60
Pro Ser Cys Leu Asp Met Glu Gly Ser Val Ser Ala Val Thr Glu Glu
65                  70                  75                  80
Ala Ala Leu Cys Ala Gly Trp Leu Ser Gln Pro Thr Pro Ala Thr Leu
                85                  90                  95
Gln Pro Leu Ala Pro Trp Thr Pro Tyr Thr Glu Tyr Val Pro His Glu
            100                 105                 110
Ala Val Ser Cys Pro Tyr Ser Ala Asp Met Tyr Val Gln Pro Val Cys
        115                 120                 125
Pro Ser Tyr Thr Val Val Gly Pro Ser Ser Val Leu Ala Tyr Ala Ser
    130                 135                 140

Pro Pro Leu Ile Thr Asn Val Thr Thr Arg Ser Ser Ala Thr Pro Ala
145                 150                 155                 160
Val Gly Pro Pro Leu Glu Gly Pro Glu His Gln Ala Pro Leu Thr Tyr
                165                 170                 175
Phe Pro Trp Pro Gln Pro Leu Ser Thr Leu Pro Thr Ser Thr Leu Gln
            180                 185                 190
Tyr Arg Pro Pro Ala Pro Ala Leu Pro Gly Pro Gln Phe Val Gln Leu
        195                 200                 205
Pro Ile Ser Ile Pro Glu Pro Val Leu Gln Asp Met Glu Asp Pro Arg
    210                 215                 220
Arg Ala Ala Ser Ser Leu Thr Ile Asp Lys Leu Leu Leu Glu Glu Glu
225                 230                 235                 240
Asp Ser Asp Ala Tyr Ala Leu Asn His Thr Leu Ser Val Glu Gly Phe
                245                 250                 255
<210> 3
<211> 19
<212> RNA
<213> Artificial Sequence
<220>
<223> Description of Artificial Sequence: Synthetic RNA
<400> 3
caccuuacac cgaguaugu                                    19
<210> 4
<211> 17
<212> RNA
<213> Artificial Sequence
<220>
<223> Description of Artificial Sequence: Synthetic RNA
<400> 4
gguucugugu cugcagu                                      17

## Description

### Technical Field

The present invention relates to a method for detecting cancer by detecting genome amplification in the human chromosome 11q23.1 region for the purpose of diagnosing multiple myeloma at early stage by observing its genotype. The present invention also relates to a method for inhibiting tumor growth based on findings concerning the correlation between the POUR domain, class 2, associating factor 1 (POU2AF1) gene and multiple myeloma.

### Background Art

Multiple myeloma (MM) is caused by abnormal growth of plasma cells originating from B cells. Currently, it has a poor long-term prognosis (MM is reported to have an average survival duration of about 3 years after initiation of treatment. There is a chance of about 10% of surviving five years, and of about 3% to 5% of surviving 10 or more years). Multiple myeloma is described in Bhawna Sirohi et al., Lancet, 363, 875-87, 2004. As association of cancer genes with this disease, such as the association of c-my and Bcl-1/2, point mutation of N-ras and K-ras, and abnormality of translocation of several IgH genes have been reported up to the present. However, a gene abnormality that is peculiar to myeloma has not yet been elucidated. As a result of research that has been undertaken in the past, cells are considered to undergo continuous genetic change during the process of differentiation and growth thereof, and to consequently undergo tumorigenesis. Since types of genetic changes that induce multiple myeloma have not yet been elucidated, there has been no method for detecting multiple myeloma or a method for typing the same that involves the use of a gene.

### Disclosure of the Invention

If the mechanism of multiple myeloma at the gene level is clarified, early detection of multiple myeloma during the process of tumorigenesis at the gene level and diagnosis of malignancy can be realized. Further, selection or development of a drug and establishment of a therapeutic method should be realized based on such mechanism. More specifically, a gene exhibiting a behavior peculiar to multiple myeloma is identified, and the identified gene is technically analyzed, which in turn results in resolution of the aforementioned issue. That is, it is an object of the present invention to identify a gene that relates to exhibition of behavior peculiar to cancer such as multiple myeloma to provide a method for detecting cancer and a cell-growth inhibitor.

The DNA microarray-based comparative genomic hybridization (CGH) technique, i.e., the array CGH technique, is a simple, rapid, and the best method for analyzing genetic abnormalities caused by amplification and deletion of many genes in genomic DNA. By selecting 800 types of BAC/PAC DNA that are to be mounted on the CGH array in order to analyze gene abnormalities in the genome associated with tumorigenesis and malignant degeneration of tumors (Takada H. et al., Cancer Sci. 96, 100-105, 2005), a cancer gene that accelerates tumorigenesis of multiple myeloma, i.e., the POU2AF1 gene, was identified successfully. The present inventors also succeeded in discovering that amplification of the POU2AF1 gene, i.e., increase in the POU2AF1 protein, remarkably accelerated multiple myeloma cell growth and that inhibition of the POU2AF1 gene transcript remarkably lowered multiple myeloma cell growth. This has led to the completion of the present invention.

Specifically, the present invention provides a method for detecting cancer which comprises detecting and/or typing tumorigenesis of a specimen by employing gene amplification in the chromosome 11 q23 region of the specimen as an indicator.

Preferably, the gene is any of the RDX, FDX1, ARHGAP20, POU2AF1, LAYN, SNF1LK2, PPP2R1B, or ALG9 genes.

Preferably, an indicator for amplification is at least 1.32 times higher than that of a normal specimen.

Preferably, the specimen is of multiple myeloma.

Preferably, the cancer is multiple myeloma.

Preferably, the present invention provides a method for detecting multiple myeloma which comprises detecting and/or typing the multiple myeloma tumorigenesis of a specimen by employing amplification of the POU2AF1 gene as an indicator.

Preferably, gene amplification is detected via a DNA chip technique, Southern blotting, Northern blotting, PCR, real-time RT-PCR, FISH method, CGH method, gene amplification, RFLP detection, nucleotide sequencing, or the array CGH method.

Another aspect of the present invention provides a method for detecting cancer which comprises detecting and/or typing tumorigenesis of a specimen using, as an indicator, the elevated expression level of any of the RDX gene, the FDX1 gene, the ARHGAP20 gene, the POU2AF1 gene, the LAYN gene, the SNF1LK2 gene, the PPP2R1B gene, or the ALG9 gene of the specimen.

Another aspect of the present invention provides a method for inhibiting cell growth which comprises introducing *in vitro* siRNA, shRNA, an antisense oligonucleotide, or a loss-of-function gene of a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene into a tumor cell.

Another aspect of the present invention provides a method for inhibiting cell growth which comprises introducing *in vitro* a loss-of-function protein of a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene into a tumor cell.

Another aspect of the present invention provides a cell growth inhibitor which comprises siRNA, shRNA, antisense oligonucleotide, or a loss-of-function gene of a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene.

Another aspect of the present invention provides a cell growth inhibitor which comprises a loss-of-function protein of a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene.

Another aspect of the present invention provides a method for activating cell growth which comprises introducing *in vitro* a gene selected from among the RDX gene, the FDX1 gene, the ARHGAP20 gene, the POU2AF1 gene, the TNFRSF17 gene, the LAYN gene, the SNF 1 LK2 gene, the PPP2R1B gene, and the ALG9 gene into a cell.

Another aspect of the present invention provides a cell growth activator which comprises a gene selected from among the RDX gene, the FDX1 gene, the ARHGAP20 gene, the FOU2AF1 gene, the TNFRSF17 gene, the LAYN gene, the SNF1LK2 gene, the PPP2R1B gene, and the ALG9 gene.

### Brief Description of the Drawings

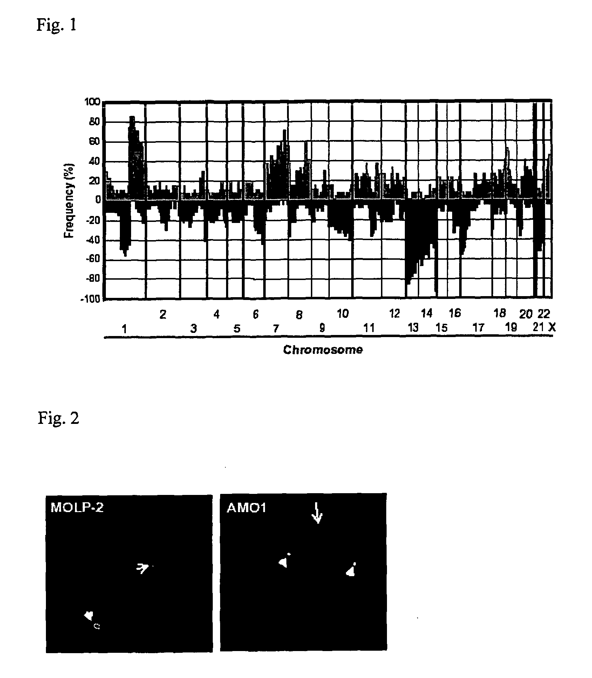

Fig. 1 shows the results of array CGH analysis of the genomic DNA samples prepared from 28 types of multiple myeloma cells using the CGH array (MGC Cancer Array-800). As a control sample, lymphocyte-derived genomic DNA obtained from a healthy volunteer was used. Frequency of abnormal cells in the amplified and deleted chromosome regions in relation to the total cell lines (28 types) were shown as chromosome positions by representing amplification (green) and deletion (red) on the vertical axis and by referring to the UCSC mapping position (http://genome.ucsc.edu/[version May, 2004]) on the horizontal axis.

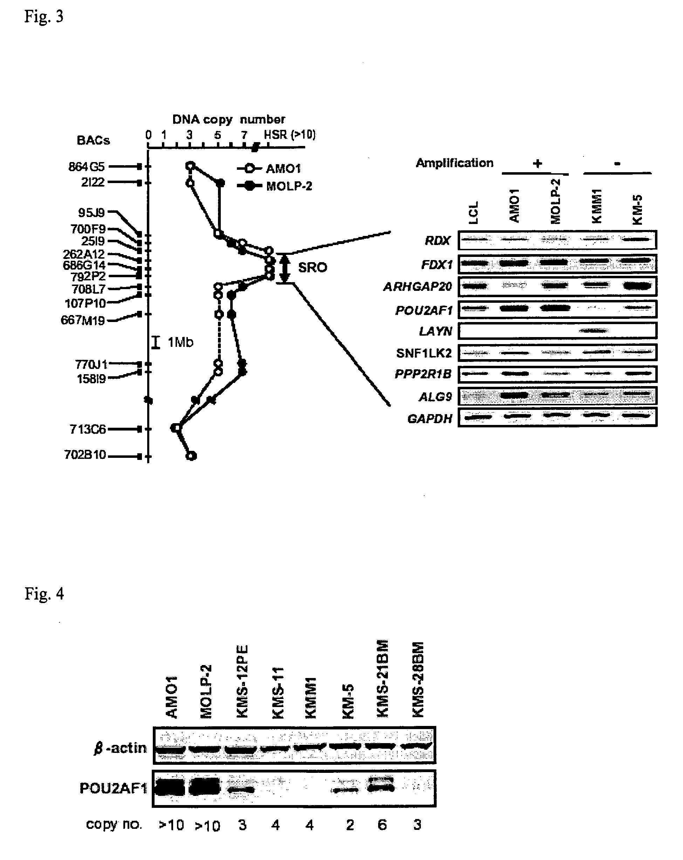

Fig. 2 shows the results of FISH analysis of BAC clones of RP11-792P2 in the chromosome 11q23 region of AMO1 and MOLP-2 cells observed by the array CGH technique. An arrow indicates a FISH signal at the original chromosome position, and a wedge shape indicates a strong amplification signal comprising multiple copies of genes, which indicates the occurrence of gene amplification.
Fig. 3 shows the results of experiments where the fluorescent signal intensities of 15 types of RP-11BAC clones in the 11q23 region were determined by FISH analysis of AMO1 and MOLP-2 cells, the copy numbers thereof in the corresponding genomic DNA regions were determined based on the fluorescent signal intensities, and the results were schematically expressed (the left portion). The vertical axis represents the BAC clone IDs in the 11q23 region and relative positions thereof. The homogeneously stained regions with the highest luminescence (i.e., the homogenously staining regions (HSRs)) were substantially the same for both cells, and the regions of interest were each determined to be an about 3-Mb (megabase) region between RP11-2519 and 708L7. The right portion of the figure shows the results of electrophoresis in 3% agarose gel of the AMO1, RDX, ARHGAP20, POU2AF1, SNF1LK2, FPP2R1B, and ALG9 genes (mRNA) located in the 11q23 region, following PCR for the purpose of simple quantification. The lowest portion shows the results of electrophoresis using GAPDH as a control example for the expression level. AMO1 and MOLP-2 cell lines in which the 11q23 regions were amplified, KMM1 and KM-5 cell lines in which the 11q23 regions were not amplified, and lymphocyte clones (LCL) obtained from healthy volunteers as controls were used.
Fig. 4 shows the expression levels of the POU2AF1 protein in 8 types of multiple myeloma cell lines which were quantified by Western blotting. The upper portion shows the results of Western blotting of β-actin, which is a control example for monitoring protein levels. In the figure, 2 types of bands observed in the POU2AF1 protein represent isoforms p34 and p35. The lower portion of the figure shows the copy number of the POU2AF1 gene in genomic DNA determined by FISH.

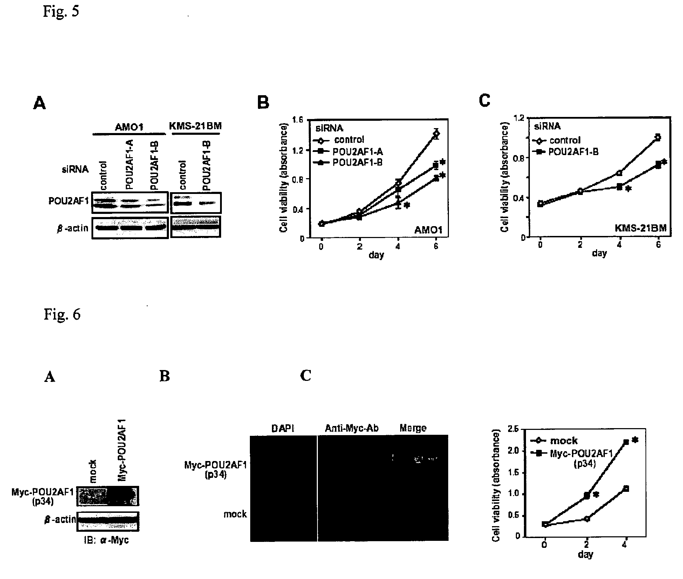

Fig. 5A shows the results of Western blotting analysis of AMO1 and KMS-21BM cells into which POU2AF1-A and POU2AF1-B, i.e., 2 siRNA species of POU2AF1, had been transduced. The lower portion of the figure shows the results of Western blotting of β-actin, which is a control example for monitoring protein levels. Fig. 5B and Fig. 5C show the results of WST assay of the viable cell count with the elapse of time by transducing siRNA of POU2AF1 into 1 x 10⁴ AMO1 and KMS-21BM cells and sowing the resultant on a 96-well plate. The experiment was carried out twice in triplicate, and the asterisk is used for results that were determined to be significant (p< 0.05) by the unpaired Student's t test.
Fig. 6A shows the result of experiment where an expression vector (pCMV-Tag3-p34-POU2AF1) which can express a full-length POU2AF1 gene (p34) with Myc being bound at the N-terminus was constructed, the resulting vector was introduced into a KMS-11 cell in which the expression level of the endogenous POU2AF1 gene was low, and drug selection was carried out for 3 weeks. Fig. 6A shows the results of detecting the conditions of POU2AF1 protein expression using a solution of disrupted cells by Western blotting with the use of an anti-Myc antibody. pCMV-3B containing no foreign gene was introduced into the KMS-11 cell, and the resultant was designated as a "mock" (negative control). Fig. 6B shows the results of fluorescence microscopic observation of the transformed cells cultured on a glass slide and stained with the anti-Myc polyclonal antibody and with the Alexa 488-labeled sheep-derived anti-rabbit antibody (Molecular Probes). The results of nuclear staining of DAPI are shown in the left portion. The hues were adjusted and represented as "merge." The lower portion shows the results concerning the mock. Fig. 6C shows the results of WST assay of the mock control and POU2AF1 gene-expressing cells by determining the cell growth rate as the viable cell count.

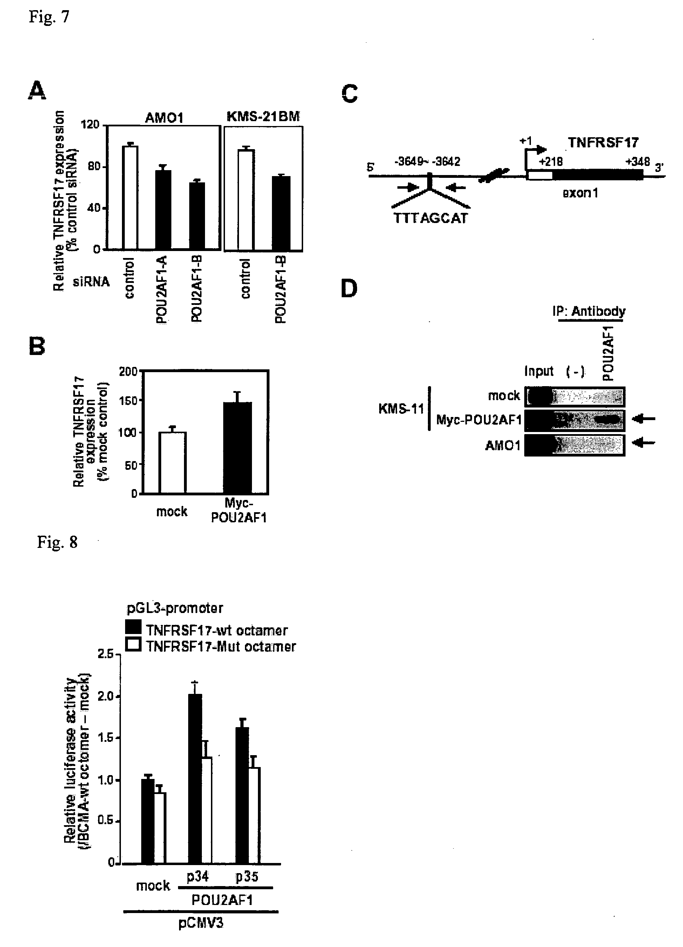

Fig. 7A shows the results of real-time RT-PCR analysis of the expression levels of the TNFRSF17 gene 48 hours after the transduction of siRNA of POU2AF1-A and POU2AF1-B into 2 x 10⁶ of AMOI cells and KMS-21BM cells (only POU2AF1-B regarding the KMS-21BM cells). The expression level of the TNFRSF17 gene in the presence of siRNA as a negative control based on random sequencing was designated as 100, and the relative amounts were represented by numerical values. Fig. 7B shows the results of comparison via real-time RT-PCR of TNFRSF17 gene expression in the POU2AF1 gene-expressing cell line established by external transduction into KMS-11 cells and in the mock control. The expression level of the TNFRSF17 gene in the mock control was designated as 100, and the expression level was represented in terms of a relative value. Fig. 7C shows the presence of an octamer site that can promote transcription of POU2AF1 from a site 3,642 bases upstream of the transcription initiation site (+1) by searching for a transcription-factor-binding site in the 5' region of the human TNFRSF17 gene in genomic DNA using a database. An arrow represents a PCR primer that detects the octamer site used for the subsequent experiment. Fig. 7D shows the results of PCR conducted in order to inspect whether or not the immune precipitate using an antibody against the POU2AF1 protein had the octamer site with reference to chromatins of the AMO1 cell and the transgenic KMS-11 cell (mock control, POU2AF1 gene-expressing cell). As indicated by an arrow in Fig. 7C, PCR primers that sandwich the octamer site were used. The solution of disrupted cells was subjected to immunoprecipitation using an anti-POU2AF1 antibody, and 1/100 of the resultant was used as a template for PCR. The term "input" indicates a chromatin material before immunoprecipitation, (-) indicates a negative control without any immunoprecipitation antibody, and an arrow indicates a detected octamer site.

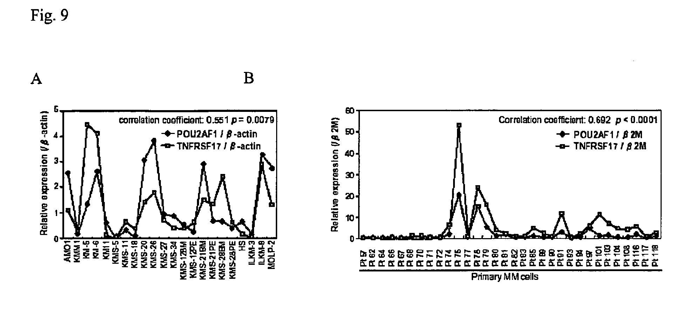

Fig. 8 shows the results of assaying luminescence by transducing a luciferase promoter-reporter vector containing wild type (wt) and mutant (Mut) octamer sites in octamer site-containing DNA regions upstream of the TNFRSF17 gene into KMS-11 cells together with the POU2AF1 isoforms p35 and p34 expression vectors. The TNFRSF17-wt octamer is designated as 1, and relative luciferase activity is shown.
Fig. 9 shows the expression levels of the POU2AF1 gene and the TNFRSF17 gene in primary cells established from the multiple myeloma cell line and from the multiple myeloma clinical sample, which were assayed by quantitative PCR. The values were represented in terms of the ratio against β-actin and β2-microglobulin (β2M) (Fig. 9A: cell line; B: primary cell). The correlation coefficient and the p value are shown in the upper-right portions of the charts.

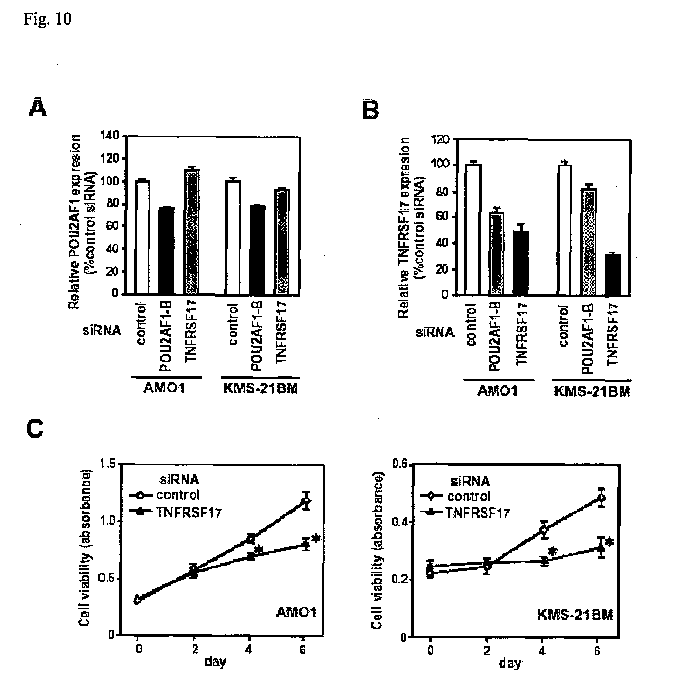

Fig. 10A and Fig. 10B show the result of the analysis wherein, with the use of the AMO1 and the KMS-21BM cells, siRNA comprising a nonspecific sequence as a control, siRNA of the POU2AF1 gene and of the TNFRSF17 gene were transduced, and the expression levels of the POU2AF1 gene and the TNFRSF17 gene were assayed by quantitative PCR using β-actin as an indicator. The determined values were represented in terms of the relative expression levels in relation to the control value (100) (Fig. 10A: POU2AF1; Fig. 10 B: TNFRSF17). Fig. 10C shows the results of WST assay of the viable cell counts in the AMO1 and in the KMS-21BM cells after the inhibition of the TNFRSF17 gene by siRNA (0, 2, 4, and 6 days later). An asterisk indicates that the measured values were significant compared with the control as a result of an unpaired Student's t test (p < 0.05).
Preferred Embodiments of the Invention

Hereafter, the present invention is described in greater detail.

### (1) Method for detecting cancer

According to the method for detecting cancer of the present invention, gene amplification in the chromosome 11 q23 region of a specimen is employed as an indicator to detect and/or type the tumorigenesis of the specimen.

Based on research that was conducted in the past, a transcript of the human POU2AF1 gene (also known as OBF-1 (OCT binding factor 1), BOB-1 (B-cell-specific coactivator OBF-1), or OCA-B) is already known. This gene is the gene in the 11q23.1 chromosome (Junker, S. et al., Genomics 33: 143-145, 1996). The nucleotide sequence of the human POU2AF1 gene is registered in the National Center for Biotechnology Information (NCBI) database as NM_006235, and the amino acid sequence of the human POU2AF1 protein is registered in the same database as NP_006226. The nucleotide sequence of the human POU2AF1 gene is as shown in SEQ ID NO: 1, the POU2AF1 protein encodes a region comprising nucleotides 524 to 1294 of the nucleotide sequence as shown in SEQ ID NO: 1, and the amino acid sequence thereof is as shown in SEQ ID NO: 2.

The term "POU2AF1 gene" used herein refers to a human-derived gene that is identified by the aforementioned nucleotide sequence, and the term "POU2AF1 protein" refers to a protein that is encoded by the POU2AF1 gene and identified by the aforementioned amino acid sequence. The protein encoded by the human POU2AF1 gene is known to have the POU domain (the POU domain protein has in its sequence a common DNA-binding sequence containing a POU specific domain and a POU homeodomain and functions as a transcription regulator), and it is known to bind to the transcription factor OCT1 or OCT2, and enhance its activity. However, the fact that this human POU2AF1 gene is an important cancer gene associated with development of human multiple myeloma was previously unknown.

As genes that are present in the chromosome 11 q23 region, the RDX gene, the FDX1 gene, the ARHGAP20 gene, the LAYN gene, the SNF1LK2 gene, the PPP2R1B gene, and the ALG9 gene are known. Such genes are registered in the National Center for Biotechnology Information (NCB1) database under the following RefSeq IDs: RDX (radixin): NM_002906; FDX1 (ferredoxin 1, a nuclear gene encoding mitochondrial protein): NM_004109; ARHGAP20 (Rho GTPase activating protein 20): NM_020809; LAYN (layilin): NM_178834; SNF1LK2 (SNF1-like kinase 2): NM_015191, PPP2R1B: two types of splice variants are present (protein phosphatase 2 (formerly 2A) and regulatory subunit A (PR 65), beta isoform (PPP2R1B), transcript variant 1 or protein phosphatase 2 (formerly 2A), regulatory subunit A (PR 65), beta isoform (PPP2R1B), transcript variant 2), registered as NM_002716 or NM_181699; and ALG9 (asparagine-linked glycosylation 9 homologue (*S*. *cerevisiae,* alpha- 1,2-mannosyltransferase)): NM_024740.

As described above, an embodiment of the method for detecting cancer of the present invention is characterized in that amplification of the human POU2AF1 gene in the multiple myeloma cell is detected. The target multiple myeloma cell for detecting amplification of the human POU2AF1 gene is preferably a viable cell recovered from a specimen donor. The cell specimen may be a bone marrow cell or blood cell of a healthy volunteer, or the tumor cell of a patient with multiple myeloma. In practice, a main target cell can be a lesion cell in which an indication of tumorigenesis is observed in bone marrow as a result of the test, or a multiple myeloma cell which is determined to be multiple myeloma wherein the degree of progress or recovery should be determined.

When amplification of the human POU2AF1 gene is observed in a "lesion cell in which an indication of tumorigenesis is observed in bone marrow as a result of the test" by the detection method of the present invention, this indicates that such lesion cell is headed toward tumorigenesis or is already in a tumorigenic state, malignancy is advancing, and a rapid and full-fledged treatment (e.g., a full-fledged chemotherapy) is required. When amplification of the human POU2AF1 gene is observed in a "multiple myeloma cell that is determined to be multiple myeloma and regarding which a method of typing MM should be determined," this also indicates that the tumor cell should be typed and an adequate full-fledged treatment (e.g., a full-fledged chemotherapy) should be provided. A multiple myeloma cell recovered as a specimen can be subjected to necessary processing, such as preparation of DNA or RNA from the recovered cell, and the resultant can be the target of the detection method.

In the present invention, amplification of the human POU2AF1 gene in the multiple myeloma cell can be detected to detect and/or type tumorigenesis of the cell, as described above.

In the present invention, gene amplification in the chromosome 11 q23 region of the specimen is employed as an indicator to detect and/or type tumorigenesis of the specimen. Types of cancer to be detected and/or typed are not particularly limited as long as gene amplification in the chromosome 11 q23 region is observed. Specific examples of cancer include, but are not limited to, malignant melanoma, malignant lymphoma, lung cancer, esophageal cancer, gastric cancer, large bowel cancer, rectal cancer, colonic cancer, ureteral tumor, gallbladder cancer, bile duct cancer, biliary tract cancer, mammary cancer, liver cancer, pancreatic cancer, testicular tumor, maxillary cancer, lingual cancer, labial cancer, oral cavity cancer, pharyngeal cancer, laryngeal cancer, ovarian cancer, uterine cancer, prostate cancer, thyroid gland cancer, brain tumor, Kaposi's sarcoma, angioma, leukemia, polycythemia vera, neuroblastoma, retinoblastoma, myeloma, bladder tumor, sarcoma, osteosarcoma, myosarcoma, skin cancer, basal cell cancer, skin appendage carcinoma, metastatic skin cancer, and cutaneous melanoma. Among them, a particularly preferable target is myeloma.

Examples of genes in the chromosome 11 q23 region that are used as indicators for amplification in the method of the present invention include the RDX gene, the FDX1 gene, the ARHGAP20 gene, the POU2AF1 gene, the LAYN gene, the SNF1LK2 gene, the PPP2R1B gene, and the ALG9 gene. When such genes are amplified in amounts at least 1.32 times, preferably at least 1.5 times, more preferably at least 2 times, still more preferably at least 3 times, further preferably at least 4 times, and particularly preferably at least 5 times higher the amount of genes obtained from healthy volunteers, such amplification can be determined to represent tumorigenesis in the method of the present invention.

Examples of representative methods for directly detecting amplification of genes in the chromosome 11 q23 region, such as the human POU2AF1 gene, include the comparative genomic hybridization (CGH) technique and the fluorescence *in situ* hybridization (FISH) technique. According to an embodiment of the detection method of the present invention, bacterial artificial chromosome (BAC) DNA, yeast artificial chromosome (YAC) DNA, or P1-derived artificial chromosome (PAC) DNA (and hereafter, it may be referred to as BAC DNA or the like) having the human POU2AF1 gene is labeled, and the amplified portion of the human POU2AF1 gene can be detected via FISH. Specific examples of BAC DNA having the human POU2AF1 gene include RP11-262A12, RP11-686G14, and RP11-792P2,

This embodiment of the method is preferably and practically carried out with the use of a substrate on which genomic DNA was immobilized.

The amount of BAC DNA or the like that is commonly obtained is too small to mass-produce substrates on which genomic DNA was immobilized and to put the same to practical use. Accordingly, such DNA must be obtained as a gene amplification product (and this process of gene amplification is also referred to as "infinuitazatlon"). In the infinitization process, BAC DNA or the like is first digested with a four-nucleotide recognition enzyme, such as RsaI, DpnI, or HaeIII, and an adaptor is added thereto to perform ligation. An adaptor is an oligonucleotide of 10 to 30 nucleotides, and preferably 15 to 25 nucleotides, and two strands each have a complementary sequence. After annealing, an oligonucleotide at the 3'-end, which is to be blunt-ended, should be phosphorylated. Subsequently, a primer having the same sequence as an oligonucleotide of the adaptor may be used to amplify DNA of interest by polymerase chain reaction (PCR) for infinitization. Alternatively, an aminated oligonucleotide of 50 to 70 nucleotides that is peculiar to BAC DNA or the like can be used as a detection probe.

The BAC DNA or the like that has been infinitized in such a manner can be immobilized on a substrate, and preferably a solid substrate, to produce a substrate of interest on which DNA has been immobilized. A solid substrate is preferably made of glass. A solid substrate such as a glass substrate is preferably coated with poly-L-lysine, aminosilane, gold/aluminum or the like via adhesion.

The infinitized DNA is spotted on a substrate at concentration of preferably 10 pg/µl to 5 µg/µl, and more preferably 1 ng/µl to 200 ng/µl. The amount to be spotted is preferably 1 nl to 1 µl, and more preferably 10 nl to 100 nl. The size and the configuration of each spot to be immobilized on a substrate are not particularly limited. For example, the diameter of a spot can be 0.01 to 1 mm, and the aerial configuration can have a circular to oval shape. The thickness of a dry spot is not particularly limited, and it is 1 to 100 µm. The number of spots is not particularly limited, and 10 to 50,000, and more preferably 100 to 5,000 spots are used per substrate. Each DNA is spotted singly to in quadruplicate, and spotting in duplicate or in triplicate is preferable.

Dry spots can be prepared by, for example, using a spotter, adding the infinitized BAC DNA or the like dropwise onto a substrate to form a plurality of spots, and drying the spots. An inkjet printer, pin array printer, or Bubble Jet^{(R)} printer may be used as a spotter, with the use of an inkjet printer being preferable. An example is GENESHOT (NGK Insulators, Ltd., Nagoya, Japan).

The BAC DNA or the like that had been infinitized in the above-described manner can be immobilized on a substrate, preferably on a solid substrate, to produce a substrate on which DNA has been immobilized.

Also, Southern blotting may be performed as a means for directly detecting amplification of the human POU2AF1 gene. Southern blotting is a technique wherein genomic DNA is separated from a specimen and immobilized, the resultant is subjected to hybridization to the human POU2AF1 gene, and the presence of the gene in the specimen is then detected by detecting hybridization. PCR can also be employed as a means for directly detecting amplification of the human POU2AF1 gene. Genomic DNA is separated from the analyte, amplified using a primer that is capable of amplifying all or part of the gene, and quantified. Thus, amplification can be detected.

Alternatively, amplification of the human POU2AF1 gene may be detected by Northern blotting or real-time RT-PCR. Northern blotting is a technique wherein mRNA obtained from a specimen is separated and immobilized, the resultant is subjected to hybridization to the human POU2AF1 gene, and the presence of mRNA in the gene of the specimen is then detected by detecting hybridization. Real-time RT-PCR is a technique wherein amplification of the target gene resulting from reverse transcription and PCR is assayed with the elapse of time (real time). According to this technique, template mRNA can be quantified based on the rate of amplification. Such quantification is performed using a fluorescent dye, and two types of techniques are known; i.e., a method involving the use of a dye (e.g., SYBR green) that emits fluorescence via intercalation in a double-stranded DNA-specific manner; and a method involving the use of a probe comprising an oligonucleotide specific to the DNA sequence to be amplified and a fluorescent dye bound thereto.

In the present invention, the elevated expression level of a gene selected from among the RDX gene, the FDX1 gene, the ARHGAP20 gene, the POU2AF1 gene, the LAYN gene, the SNF1LK2 gene, the PPP2R1B gene, and the ALG9 gene of the specimen is employed as an indicator to detect and/or type tumorigenesis of the specimen. Thus, cancer can be detected.

In the present invention, as an example, an antibody against the POU2AF1 protein or a fragment thereof is used to analyze the POU2AF1 protein level in the specimen sample, and the elevated expression level of the POU2AF1 gene is employed as an indicator to detect and/or type tumorigenesis of the specimen. Thus, cancer can be detected. The expression of genes other than the POU2AF1 gene can also be analyzed in the same manner with the use of an antibody against a protein encoded by such gene or a fragment thereof. Hereafter, detection and/or typing in which the elevated expression level of the POU2AF1 gene is employed as an indicator is described as an example.

An antibody against the POU2AF1 protein that can be employed in the method of the present invention (hereafter referred to as a "POU2AF1" antibody) can be prepared in accordance with a conventional technique using all or part of the POU2AF1 protein as an antigen. "Part of the POU2AF1 protein" refers to a polypeptide comprising at least 6 continuous amino acid residues, preferably at least about 8 to 10 amino acid residues, and more preferably at least about 11 to 20 amino acid residues of the amino acid sequence of the POU2AF1 protein as shown in SEQ ID NO: 2. All or part of the antigen POU2AF1 protein may be prepared via a biological method or chemical syntheses.

A polyclonal antibody can be prepared by, for example, thoroughly immunizing an animal such as a mouse, guinea pig, or rabbit by inoculating such animal with the above antigen several times subcutaneously, intramuscularly, intraperitoneally, or intravenously, sampling blood from the immunized animal, and separating blood serum therefrom. A monoclonal antibody can be prepared by, for example, preparing a hybridoma obtained by cell fusion between the spleen cell of the mouse immunized with the above antigen and a commercialized mouse myeloma cell, culturing the hybridoma, and obtaining a monoclonal antibody from the culture supernatant of the hybridoma or ascites of a mouse to which the hybridoma has been administered.

With the use of the POU2AF1 protein antigen prepared in the above manner or a fragment thereof, the expression level of the FOU2AF1 protein in the specimen sample can be determined. Immunological assay techniques such as immunoblotting, enzyme immunoassay (EIA), radioimmunoassay (RIA), a fluorescence antibody technique, or immunocyte staining or Western blotting can be employed, for example. A fragment of the POU2AF1 protein antibody refers to a single-stranded antibody fragment (scFv) of such antibody, for example. Examples of a specimen sample that can be used include a bone marrow sample, tissue slice, blood, lymph, sputum, lung lavage, urine, stool, and tissue culture supernatant obtained from a subject suspected of having a tumor.

### (2) Method for inhibiting cell growth and cell growth inhibitor

The present invention provides a method for inhibiting cell growth which comprises introducing *in vitro* the siRNA, shRNA, antisense oligonucleotide, or a loss-of-function gene of a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene into a tumor cell, and a cell growth inhibitor which comprises the aforementioned siRNA, shRNA, antisense oligonucleotide, or loss-of-function gene. Further, the present invention provides a method for inhibiting cell growth which comprises introducing *in vitro* a loss-of-function protein of a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene into a tumor cell, and a cell growth inhibitor which comprises the aforementioned loss-of-function protein.

siRNA is double-stranded RNA comprising about 20 (e.g., about 21 to 23 nucleotides) or fewer nucleotides. Such siRNA may be expressed in the cell to inhibit expression of the target gene of siRNA (the RDX gene, the FDX1 gene, the ARHGAP20 gene, the POU2AF1 gene, the LAYN gene, the SNF1LK2 gene, the PPP2R1B gene, and the ALG9 gene in the present invention).

In the present invention, siRNA of any configuration may be used as long as it can induce RNAi. The term "siRNA" used herein is an abbreviation of "short interfering RNA," which is 10-bp or longer short double-stranded RNA that has been chemically or biochemically synthesized, that has been synthesized *in vivo,* or that is an *in vivo* degradation product of double-stranded RNA comprising at least about 40 nucleotides. In general, siRNA has a 5'-phosphoric acid or 3'-OH structure, and the 3'-end thereof protrudes by about 2 nucleotides. A protein specific to this siRNA is bound thereto in order to form an RNA-induced-silencing-complex (RISC). This complex recognizes and binds to mRNA having the same sequence as that of siRNA and cleaves mRNA at the center of siRNA with the aid of RNasaIII-like enzyme activity.

The siRNA sequence is preferably 100% consistent with the mRNA sequence, which is the target of cleavage. If nucleotides other than those at the center of siRNA are not consistent, however, RNAi-induced cleavage activity often partially remains. Accordingly, 100% consistency is not always required.

Preferably, a homologous region between the nucleotide sequence of siRNA and the nucleotide sequence of the RDX gene, the FDX1 gene, the ARHGAP20 gene, the POU2AF1 gene, the LAYN gene, the SNF1LK2 gene, the PPP2R1B gene, and the ALG9 gene e, the expression of which should be inhibited, does not contain a translation initiation region of such gene. This is because siRNA may not effectively bind to mRNA and the effects of such siRNA may be deteriorated due to the binding of various transcription factors or translation factors to the translation initiation region. Accordingly, a homologous sequence is preferably 20, and more preferably 70, nucleotides away from the translation initiation region of the gene. An example of a homologous sequence is a sequence around the 3'-end of the gene.

According to another embodiment of the present invention, shRNA (short hairpin RNA) having a short hairpin structure and a protrusion at the 3' end can be used as a factor that can inhibit expression of the target gene via RNAi. "shRNA" is a single-stranded RNA molecule of about 20-bp or longer (typically 21 to 23 nucleotides, for example) that has a double-strand structure therein and a hairpin-like structure due to a nucleotide sequence that is a partial palindrome. Such shRNA is introduced into the cell, it is degraded into a length of about 20 nucleotides (typically, for example, 21 to 23 nucleotides) in the cell, and it is capable of inducing RNAi, as in the case of siRNA. Since shRNA induces RNAi, as in the case of siRNA as described above, shRNA can be effectively used in the present invention.

Preferably, shRNA has a 3'-protruding end. The length of the double-stranded portion is not particularly limited, and it is preferably about 10 nucleotides or longer, and more preferably about 20 nucleotides or longer. The 3'-protruding end is preferably DNA, more preferably DNA of at least 2 nucleotides, and still more preferably DNA of 2 to 4 nucleotides.

In the present invention, siRNA or shRNA can be used as a factor that can inhibit expression of the RDX gene, the FDX1 gene, the ARHGAP20 gene, the POU2AF1 gene, the LAYN gene, the SNF1LK2 gene, the PPP2R1B gene, and the ALG9 gene via RNAi, as described above. Examples of advantages of siRNA include: (1) even when it is introduced into a cell, RNA is not incorporated into a chromosome of a normal cell, variation that is inherited by the progeny does not occur, and thus the treatment is highly safe; and (2) short double-stranded RNA is relatively easily chemically synthesized, and a double-strand is more stable. An advantage of shRNA is, for example, the fact that when treatment is carried out by inhibiting gene expression for a long period of time, a vector that transcribes shRNA in the cell may be prepared and introduced into the cell.

siRNA or shRNA that can inhibit expression of the RDX gene, the FDX1 gene, the ARHGAP20 gene, the POU2AF1 gene, the LAYN gene, the SNF1LK2 gene, the PPP2R1B gene, and the ALG9 gene via RNAi used in the present invention may be artificially and chemically synthesized. Alternatively, a DNA sequence of a sense strand and that of an antisense strand are invertedly ligated in order to form hairpin DNA, and RNA is synthesized *in vitro* from such DNA with the use of T7 RNA polymerase. Thus, siRNA or shRNA can be prepared. When siRNA or shRNA is synthesized *in vitro,* antisense and sense RNA can be synthesized from template DNA using T7 RNA polymerase and a T7 promoter. After such RNAs are annealed *in vitro,* they are introduced into cells, RNAi is induced, and expression of the target genes is inhibited. Such RNA can be introduced into the cell by the calcium phosphate method or with the use of various transfection reagents (e.g., oligofectamine, lipofectamine, or lipofection), for example.

The aforementioned siRNA or shRNA is useful as a cell growth inhibitor. The cell growth inhibitor of the present invention can be administered via oral administration, parenteral administration (e.g., intravenous, intramuscular, subcutaneous, intracutaneous, transmucosal, intrarectal, or intravaginal administration, topical administration to a lesion, and skin administration), or direct administration to a lesion. When the agent of the present invention is used as a pharmaceutical composition, a pharmacologically acceptable additive can be incorporated according to need. Specific examples of a pharmacologically acceptable additive include, but are not limited to, an antioxidant, a preservative, a coloring agent, a flavoring agent, a diluent, an emulsifier, a suspending agent, a solvent, a filler, an extender, a buffer, a delivery vehicle, an attenuant, a carrier, an excipient, and/or a pharmacological adjuvant.

The dosage form of the agent of the present invention is not particularly limited. Examples thereof include a solution, an injection, and a controlled-release agent. An aqueous or non-aqueous solvent may be used to formulate the agent of the present invention as the aforementioned pharmaceutical preparation.

Further, siRNA and shRNA, which are active ingredients of the cell growth inhibitor of the present invention, can be administered in the form of non-viral or viral vectors. In the case of a non-viral vector, for example, a method wherein a nucleic acid molecule is introduced using a liposome (e.g., the liposome, HVJ-liposome, cationic liposome, lipofection, or lipofectamine method), microinjection, or a method wherein a nucleic acid molecule is introduced into the cell with a carrier (metal particles) by the gene gun method may be employed. When siRNA or shRNA is administered to a living organism using a viral vector, a viral vector such as a recombinant adenovirus or retroviral vector can be used. DNA that expresses siRNA or shRNA is introduced into a DNA or RNA of a virus such as detoxicated retrovirus, adenovirus, adeno-associated virus, herpes virus, vaccinia virus, poxvirus, poliovirus, Sindbis virus, hemagglutinating virus of Japan, or SV40, and the cell or tissue is infected with the recombinant virus. Thus, a gene can be introduced into the cell or tissue.

A person skilled in the art can determine the dose of the cell growth inhibitor of the present invention in accordance with the purpose of use, the severity of the disease, the age, the body weight, sex, or disease history of the patient, or the type of siRNA or shRNA used as an active ingredient. The dose of siRNA or shRNA is not particularly limited. For example, it is about 0.1 ng to about 100 mg/kg/day, and preferably about 1 ng to about 10 mg/kg/day. In general, the effect of RNAi is observed 1 to 3 days after administration. Accordingly, administration once every day to once every 3 days is preferable. When an expression vector is used, administration can be carried out about once a week.

In the present invention, an antisense oligonucleotide can be used as a cell growth inhibitor. The antisense oligonucleotide used in the present invention is a nucleotide that is complementary to or hybridizes to a sequence comprising 5 to 100 continuous nucleotides in the DNA sequence of the RDX gene, the FDX1 gene, the ARHGAP20 gene, the POU2AF1 gene, the LAYN gene, the SNF1LK2 gene, the PPP2R1B gene, and the ALG9 gene. It may be DNA or RNA, and it may be modified as long as no interference with the functions thereof takes place. The term "antisense oligonuc1eotjde" used herein refers not only to a sequence comprising nucleotides complementary to nucleotides constituting a given region of DNA or mRNA but also a sequence comprising several mismatches, as long as DNA or mRNA can stably hybridize to an oligonucleotide.

The antisense oligonucleotide may be modified. By providing adequate modification, such antisense oligonucleotide becomes less likely to be decomposed *in vivo,* and it can more stably inhibit the target gene. Examples of such modified antisense oligonucleotides include those of S-oligo (phosphorothioate), C-5 thiazole, D-oligo (phosphodiester), M-oligo (methylphosphonate), peptide nucleic acid, phosphodiester bond, C-5 propinyl pyrimidine, 2-0-propyl ribose, and 2'-methvxyethoxy ribose types. In the antisense oligonucleotide, at least some oxygen atoms that constitute a phosphoric acid group may be substituted with sulfur atoms and modified. Such antisense oligonucleotide is excellent particularly in nuclease resistance, water solubility, and affinity with RNA. An example of the antisense oligonucleotide in which at least some oxygen atoms that constitute a phosphoric acid group may be substituted with sulfur atoms and modified is an S-oligo type oligonucleotide.

The number of nucleotides of the antisense oligonucleotide is preferably 50 or less, and more preferably 25 or less. If the number of nucleotides becomes excessively large, oligonucleotide synthesis becomes laborious and incurs an elevated cost, which in turn lowers the yield. Further, the number of nucleotides of the antisense oligonucleotide is preferably 5 or more, and more preferably 9 or more. When the number of nucleotides is 4 or less, specificity for the target gene is disadvantageously lowered.

An antisense oligonucleotide (or a derivative thereof) can be synthesized in accordance with a common technique. For example, an antisense oligonucleotide can be easily synthesized using a commercially available DNA synthesizer (e.g., a DNA synthesizer manufactured by Applied Biosystems). An antisense oligonucleotide can be synthesized by, for example, a solid-phase synthesis method using phosphoroamidite or a solid-phase synthesis method using hydrogen phosphonate.

When an antisense oligonucleotide is used as a cell growth inhibitor in the present invention, such cell growth inhibitor is administered in the form of a pharmaceutical composition comprising an antisense oligonucleotide and a pharmaceutical additive (e.g., a carrier or excipient), in general. An antisense oligonucleotide can be administered to mammalians including humans as a medicine. The route of antisense oligonucleotide administration is not particularly limited, and it may be oral or parenteral administration (e.g., intramuscular, intravenous, subcutaneous, or intraperitoneal administration, application to the mucosa of the nasal cavity or the like, or inhalation administration).

The dosage form of an antisense oligonucleotide is not particularly limited. Examples of dosage forms for oral administration include tablets, capsules, subtle granules, powder, granules, liquid drugs, and syrups. Examples of dosage forms for parenteral administration include injections, drops, suppositories, inhalants, transmucosal absorbents, transdermal absorbents, nasal drops, and ear drops. Persons skilled in the art can adequately select the form of a drug containing an antisense oligonucleotide, a pharmaceutical additive to be used, a method for preparing a drug, and the like.

The dose of an antisense oligonucleotide can be adequately determined by comprehensively taking factors such as the sex, age, or body weight of a patient, severity of condition, purpose of administration such as preventive or therapeutic administration, or the occurrence of other complications into consideration. The dose is generally 0.1 µg/kg body-weight/day to 100 mg/kg body-weight/day, and preferably 0.1 µg/kg body-weight/day to 10 mg/kg body-weight/day.

In the present invention, a loss-of-function gene of the RDX gene, the FDX1 gene, the ARHGAP20 gene, the POU2AF1 gene, the LAYN gene, the SNF1LK2 gene, the PPP2R1B gene, and the ALG9 gene can be used as a cell growth inhibitor. The term "loss-of-function gene" refers to a gene into which a variation has been introduced so as to eliminate functions of the gene. More specifically, the term refers to a gene that translates a protein that lacks its inherent functions and is generally referred to as mutein, such as a gene that lacks at least 1 constitutive amino acid of the amino acid sequence prepared from the gene of interest, a gene in which at least 1 constitutive amino acid of the gene of interest has been substituted with another amino acid, or a gene in which at least 1 amino acid added to the gene of interest.

When the loss-of-function gene is used as a cell growth inhibitor, the aforementioned gene as an active ingredient may be mixed with a base material that is commonly used for a gene therapeutic agent to prepare a cell growth inhibitor. When such gene is incorporated into a viral vector, viral particles containing recombinant vectors are prepared, and the resultant is mixed with a base material that is commonly used for a gene therapeutic agent.

As such base material, a base material that is commonly used for an injection can be used. For example, distilled water, a salt solution of sodium chloride or a salt solution mixture of sodium chloride and an inorganic salt, a solution of mannitol, lactose, dextran, or glucose, an amino acid solution of glycine or arginine, or a mixed solution of an organic acid solution or salt solution and a glucose solution can be used. Alternatively, in accordance with a method known to persons skilled in the art, an adjuvant such as an osmotic regulator, a pH adjuster, vegetable oil, or a surfactant may be added to such base material to obtain an injection in the form of a solution, suspension, or dispersion. Such injection can also be prepared as a preparation to be dissolved before use via pulverization or lyophilization.

The dosage form of the loss-of-function gene may be systemic administration, such as general intravenous or intraarterial administration, or topical administration, such as topical injection or oral administration. Further, administration can be carried out in combination with catheterization, gene introduction, surgery, or the like.

The administration route of the loss-of-function gene varies in accordance with the age, sex, condition of a patient, route of administration, the number of times of administration, or dosage form. In general, the dose is about 1 µg/kg body-weight to 1,000 mg/kg body-weight, and preferably 10 µg/kg body-weight to 100 mg/kg body-weight, per day per adult in terms of the weight of a recombinant gene. The number of times of administration is not particularly limited.

The gene therapeutic agents of the present invention described above can also be prepared by adding genes to a liposome suspension prepared in accordance with a conventional technique, lyophilizing the mixture, and thawing the mixture. Liposomes can be prepared by, for example, thin-film agitation, ultrasonication, reversed-phase evaporation, or a surfactant-removing method. Preferably, a liposome suspension is first subjected to ultrasonication, and the genes are then added thereto, from the viewpoint of improving the efficiency for sealing genes. The liposomes comprising genes sealed therein can be intravenously administered in that state or in the form of a suspension in water or physiological saline.

In the present invention, a loss-of-function protein of a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene can be used as a cell growth inhibitor (i.e., a protein preparation). When a loss-of-function protein of the POU2AF1 gene is used as a cell growth inhibitor, for example, a loss-of-function protein of POU2AF1 as an active ingredient or a loss-of-function homologous protein thereof can be administered in the form of a pharmaceutical composition containing a pharmaceutical additive (e.g., a carrier or excipient).

The dosage forms of such protein preparations are not particularly limited. Examples of preparations for oral administration include tablets, capsules, subtle granules, powder, granules, liquid drugs, and syrups. Examples of preparations for parenteral administration include injections, drops, suppositories, inhalants, transmucosal absorbents, and transdermal absorbents.

The route of such protein preparation administration is not particularly limited, and examples thereof include oral administration and parenteral administration (e.g., intramuscular administration, intravenous administration, subcutaneous administration, intraperitoneal administration, application to the mucosa of the nasal cavity or the like, or inhalation administration).

The dose of the protein preparation varies in accordance with age, sex, condition of a patient, route of administration, the number of times of administration, or dosage form. In general, the dose is about 0.001 µg/kg body-weight to 1,000 µg/kg body-weight, and preferably about 0.001 µg/kg body-weight to 100 µg/kg body-weight, per day per adult. The number of times of administration is not particularly limited.

The cell growth inhibitor of the present invention can be used for inhibiting tumors by administering an effective amount thereof to mammalians including humans. The antitumorigenic agent can be used for preventing and/or treating tumors by administering a preventively and/or therapeutically effective amount thereof to mammalians including humans.

### (3) Method for activating cell growth and cell growth activator

The present invention further provides a method for activating cell growth which comprises introducing *in vitro* a gene selected from among the RDX gene, the FDX1 gene, the ARHGAP20 gene, the POU2AF1 gene, the TNFRSF17 gene, the LAYN gene, the SNFILK2 gene, the PPP2RIB gene, and the ALG9 gene into a cell, and a cell growth activator comprising such gene.

When the POU2AF1 gene is handled, cDNA obtained from a cultured cell in accordance with a technique known in the art or cDNA enzymatically synthesized via PCR based on the nucleotide sequence as shown in SEQ ID NO: 1 of the description of the present application may be used, When DNA having the nucleotide sequence as shown in SEQ ID NO: 1 is obtained via PCR, PCR is carried out using a human chromosome DNA or cDNA library as a template and a primer designed to be capable of amplifying the nucleotide sequence as shown in SEQ ID NO: 1. The PCR-amplified DNA, fragment can be cloned into an adequate vector that is capable of amplification in an E. *coli* host or the like.

Methods for preparing a detection probe or primer for the POU2AF1 gene and for cloning the target gene are known in the art. For example, such procedures can be implemented in accordance with a method described in Molecular Cloning: A laboratory Manual, 2nd Ed., Cold Spring Harbor Laboratory, Cold Spring Harbor, NY, 1989, Current Protocols in Molecular Biology, Supplement 1 to 38, John Wiley & Sons (1987-1997), or the like.

When genes other than the POU2AF1 gene are handled, the same procedures as with the case of the POU2AF1 gene may also be carried out.

A gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene may be incorporated into a vector and may then be used in the form of a recombinant vector. A viral vector or an expression vector for an animal cell may be used, and the use of a viral vector is preferable. Examples of viral vectors include retrovirus vector, adenovirus vector, adeno-associated virus vector, baculovirus vector, vaccinia virus vector, and lentiviral vector. Use of a retroviral vector is particularly preferable for the following reasons. That is, after a host cell is infected with a retroviral vector, a virus genome is incorporated into a host cell chromosome, and a foreign gene incorporated into the vector may be expressed stably for a long period of time.

Examples of an expression vector for an animal cell include pCXN2 (Gene, 108, 193-200, 1991), PAGE207 (JP Patent Publication (Kokai) No. 6-46841 A (1994)), and a modified form of either thereof.

The aforementioned recombinant vector can be produced by introducing a vector into an adequate host for transformation and culturing the resulting transformant. When a recombinant vector is a viral vector, a host cell into which such vector is to be introduced is an animal cell that is capable of virus production. Examples thereof include COS-7 cell, CHO cell, BALB/3T3 cell, and HeLa cell. Examples of host cells for a retroviral vector include ΨCRE, ΨCRIP, and MLV. An example of a host cell for an adenoviral vector and an adeno-associated virus is the 293 cell obtained from the human embryonic kidney cell. A viral vector can be introduced into an animal cell by the calcium phosphate method, for example. When a recombinant vector is an expression vector for an animal cell, the *E*. *coli* K12 strain, HB101 strain, or DH5α strain can be used as a host cell into which such vector is to be introduced. A method of *E. coli* transformation is known in the art.

The obtained transformants are cultured in a medium under culture conditions that are suitable for each transformant. For example, *E*. *coli* transformants can be cultured in a liquid medium (pH: about 5 to 8) containing a carbon source, a nitrogen source, inorganic matter, and other substances that are necessary for growth. In general, culture is conducted at 15°C to 43°C for about 8 to 24 hours. In such a case, a recombinant vector of interest can be obtained by a common DNA isolation/purification technique after the completion of culture.

Animal cell transformants can be cultured in medium such as a 199 medium, MEM medium, or DMEM medium containing about 5% to 20% fetal bovine serum, for example. The pH level of a medium is preferably about 6 to 8. In general, culture is conducted at about 30°C to 40°C for about 18 to 60 hours. In such a case, virus particles containing recombinant vectors of interest are released in a culture supernatant. Thus, the recombinant vectors of interest can be obtained by concentrating and purifying the virus particles by cesium chloride centrifugation, polyethylene glycol precipitation, filter-concentration, or the like.

The cell growth activator of the present invention can be produced by mixing the aforementioned gene or a homologous gene thereof as an active ingredient with a base material that is usually used for a gene therapeutic agent. When the aforementioned gene or a homologous gene thereof is incorporated into a viral vector, viral particles containing a recombinant vector are prepared, and such viral particles are mixed with a base material that is commonly used for a gene therapeutic agent.

The term "homologous gene" used herein refers to: a gene having a nucleotide sequence derived from the nucleotide sequence of a given gene by deletion, addition, or substitution of 1 to several nucleotides; or a gene having a nucleotide sequence hybridizing under stringent conditions to the nucleotide sequence of a given gene. In the present invention, the term "homologous gene" preferably refers to a gene having a nucleotide sequence encoding a protein having tumorigenic activity. A homologous gene of a given gene includes a fragment of such gene.

In the "nucleotide sequence derived from the nucleotide sequence of a given gene by deletion, addition, or substitution of 1 to several nucleotides," the range of "1 to several" is not particularly limited. For example, it refers to 1 to 60, preferably 1 to 30, more preferably 1 to 20, further preferably 1 to 10, and particularly preferably 1 to 5 nucleotides.

The "gene having a nucleotide sequence derived from the nucleotide sequence of a given gene by deletion, addition, or substitution of 1 to several nucleotides" can be prepared by any techniques known in the art, such as chemical synthesis, genetic engineering, or mutagenesis. Specifically, the aforementioned given gene is used, mutation is introduced into DNA of such gene, and the nucleotide sequence of interest can be obtained. For example, the aforementioned given gene can be subjected to a method wherein the gene is contacted with a mutagenic agent, a method wherein the gene is irradiated with ultraviolet rays, or genetic engineering. A genetic engineering technique, i.e., site-directed mutagenesis, is a useful technique since this technique enables introduction of a specific mutation into a specific site. Such technique can be implemented in accordance with a method described in, for example, Molecular Cloning: A laboratory Manual, 2nd Ed., Cold Spring Harbor Laboratory, Cold Spring Harbor, NY., 1989, or Current Protocols in Molecular Biology, Supplement 1 to 38, John Wiley &Sons, 1987-1997.

The term "a nucleotide sequence hybridizing under stringent conditions" refers to a nucleotide sequence of DNA obtained by colony hybridization, plaque hybridization, or Southern hybridization using DNA as a probe. An example thereof is DNA that can be identified by performing hybridization using a filter on which colony- or plaque-derived DNA or a fragment thereof is immobilized, in the presence of 0.7 to 1.0 M NaCl at 65°C, and washing the filter with a 0.1 to 2× SSC solution (1× SSC solution comprises 150 mM sodium chloride and 15 mM sodium citrate) at 65°C. Hybridization can be performed in accordance with a method described in, for example, Molecular Cloning: A laboratory Manual, 2nd Ed., Cold Spring Harbor Laboratory, Cold Spring Harbor, NY., 1989.

An example of DNA hybridizing under stringent conditions is DNA having homology of a given level or higher to the nucleotide sequence of DNA that is used as a probe. For example, DNA having 70% or higher, preferably 80% or higher, more preferably 90% or higher, further preferably 93% or higher, and particularly preferably 95% or higher homology may be used.

The above "gene having a nucleotide sequence hybridizing under stringent conditions to the nucleotide sequence of a given gene" can be obtained under certain hybridization conditions by colony hybridization, plaque hybridization, or Southern hybridization, as described above.

In order to mix the above-mentioned gene or a homologous gene thereof as an active ingredient, a base material that is usually used for injections can be used. For example, distilled water, a salt solution of sodium chloride or a salt solution mixture of sodium chloride and an inorganic salt, a solution of mannitol, lactose, dextran, or glucose, an amino acid solution of glycine or arginine, or a mixed solution of an organic acid solution or salt solution with a glucose solution can be used. Alternatively, in accordance with a method known to persons skilled in the art, an adjuvant such as an osmotic regulator, a pH adjuster, vegetable oil, or a surfactant may be added to such base material to obtain an injection in the form of a solution, suspension, or dispersion. Such injection can also be prepared as a preparation to be dissolved before use via pulverization or lyophilization.

The administration route of the cell growth activator of the present invention may be systemic administration, such as general intravenous or intraarterial administration, or topical administration, such as topical injection or oral administration. Further, administration of the cell growth activator can be carried out in combination with catheterization, gene introduction, surgery, or the like.

The dose of the cell growth activator of the present invention varies in accordance with the age, sex, condition of a patient, route of administration, the number of times of administration, or dosage form. In general, the dose is about 1 µg/kg body-weight to 1,000 mg/kg body-weight, and preferably about 10 µg/kg body-weight to 100 mg/kg body-weight, per day per adult in terms of the weight of a recombinant gene. The number of times of administration is not particularly limited.

The present invention is hereafter described in greater detail with reference to the following examples, although the technical scope of the present invention is not limited to these examples.

### Examples

### Materials for experiment:

28 types of multiple myeloma-derived cell lines which were used include AMO1, KMM1, KM-1, KM-4, KM-5, KM-6, KM-7, KM-11, KMS-5, KMS-11, KMS-18, KMS-20, KMS-26, KMS-27, KMS-34, KMS-12BM, KMS-12PE, KMS-21BM, KMS-21PE, KMS-28BM, KMS-28PE, HS, ILKM-10, ILKM-12, ILKM-13, MOLP-2, MOLP-6, OPM-2, which were established from a clinical sample. Also, one type of lymphocyte-derived cell line obtained from healthy volunteers, which was transformed with Epstein-Barr virus was used. These cell lines were cultured in RPMI-1640 containing 10% fetal bovine serum. Bone marrow samples of 32 clinical subjects were obtained from the Nagoya City University Hospital, and these samples were used upon approval of the relevant patients and approval of the ethical committee of the hospital. At the time of diagnosis, multiple-myeloma-derived cells were selected via positive selection using the anti-CD138 antibody beads-based automatic magnetic cell sorting system (Miltenyi Biotec).

### Example 1: Amplification and deletion of human gene region in multiple myeloma cells

In order to detect novel gene amplification in multiple myeloma cells, genomic DNA prepared from 28 types of multiple myeloma cells were subjected to CGH array analysis using a CGH array of the MGC Cancer Array-800. As a control sample, Cy5-labeled lymphocyte-derived genomic DNA obtained from a healthy volunteer was used. As an analyte DNA, Cy3-labeled genomic DNA prepared from the aforementioned multiple myeloma cell was used. Specifically, DpnII-digested genomic DNA (0.5 µg) was labeled using the BioPrime Array CGH Genomic Labeling System (Invitrogen) in the presence of 0.6 mM dATP, 0.6 mM dTTP, 0.6 mM dGTP, 0.3 mM dCTP and 0.3 mM Cy3-dCTP (multiple myeloma cell), or 0.3 mM Cy5-dCTP (normal cell). Cy3- and Cy5-labeled dCTP was obtained from Amersham Biosciences. Both labeled genomic DNA samples were precipitated with the addition of ethanol in the presence of Cot-1 DNA (Invitrogen), and the resultant was dissolved in 120 µl of a hybridization mixture (50% formamide, 10% dextran sulfate, 2x SSC (1x SSC: 150 mM NaCl/15 mM sodium citrate), and 4% sodium dodecyl sulfate, pH 7.0). The resultant was incubated at 37°C for 30 minutes, applied to the CGH array mounted in a hybridization chamber, and then incubated for 48 to 72 hours while shaking at 37°C at 3 rpm (rounds per minute). Thereafter, the CGH array was washed in a 50% formamide/2x SSC (pH 7.0) solution at 50°C for 15 minutes and then washed in 2x SSC/0.1% SDS at 50°C for 15 minutes. After air-drying, the CGH array was applied to the GenePix 4000B scanner (Axon Instruments, CA, U.S.A.) to monitor fluorescence derived from Cy3 and Cy5. The results were analyzed using the GenePix Pro 6.0 imaging software (Axon Instruments, CA, U.S.A.). The average fluorescence intensity derived from Cy3 and the average fluorescence intensity derived from Cy5 were adjusted to the same value, and the quantitative change in array CGH of each gene was detected by determining the Cy3:Cy5 ratio. When the genome is free of abnormalities, the ratio is 1:1. The gene exhibiting a ratio that deviates by 0.4 or more in both positive and negative directions in terms of the log value base of 2 was designated as having an abnormality (i.e., amplification or deletion). Concerning abnormalities in amplification and deletion of chromosome regions, frequency of abnormal cells in relation to the total cell lines were shown as chromosome positions by representing amplification (green) and deletion (red) on the vertical axis and by referring to the UCSC mapping position (http://genome.ucsc.edu/[version May, 2004]) on the horizontal axis (Fig. 1). Amplification with high frequency of 50% or higher was observed in 1q, 7q, and 8q, and deletion was observed in 1p, 13q, 14q, 17p, and 22q. Deletion of the maximal frequency was observed in 14q32, and such deletion resulted from translocation of many immunoglobulin-heavy constant gamma 1 genes (IGHG1) observed in the case of leukemia or myeloma.

Further classification was made: i.e., high level amplification (log2 ratio> 2.0) and homologous deletion (log2 ratio < -2.0). Genes that had significantly changed were shown together with cells observed (Table 1).

**Table 1: Genes exhibiting high level amplification in multiple myeloma cell lines (log2 ratio > 2.0) and homologous deletion (log2 ratio < -2.0) observed by array CGH analysis (MCG Cancer Array-800)**

| BAC | Locus^{a} | | Cell line | | |
|---|---|---|---|---|---|
| | Chr. Band | Position of BAC/gene | n | Name | Gene/marker^{b} |
| **Amplification** | | | | | |
| RP11-54A4 | 1q21.2 | Chr1:147258287-147441282 | 1 | AMO1 | *MCL1* |
| RP11-316M1 | 1q21.2 | Chr1:147845228-147854045 | 1 | AMO1 | *AFIO* |
| RP11-434B12 | 2p25.1 | Chr2:8638083-8821990 | 1 | AMO1 | *ID2* |
| RP11-2026822 | 2p24.1 | Chr2:20308808-20458683 | 1 | AMO1 | *SDC1* |
| BAC-ABL1 | 2p34.12 | Chr9:128986497-129039105 | 1 | KMS-5 | *ABL1* |
| RP11-544A12 | 2p34.13 | Chr9:130994804-131191826 | 1 | KMS-6 | *CAN* |
| RP11-792P2 | 11q23.1 | Chr11;110728191-110755627 | 2 | AMO1, MOLP-2 | *POU2AF1* |
| RP11-108O10 | 11023.1 | Chr11:1110965148-111276166 | 2 | AMO1, MOLP-2 | *PPP2R1B* |
| RP11-295I5 | 12p12.1 | Chr12:25121735-25317791 | 1 | KMS-28 | *KRAS2* |
| RP11-263G6 | 12012,1 | Chr12:26122382-26326610 | 1 | KMS-26 | *KRAO* |
| RP11-993B23 | 12p12.1 | Cnr12:28002283-28014161 | 1 | KMS-26 | *PTHLH* |
| RP11-651L9 | 17p11.12 | Chr17:15875983-16059570 | 1 | KMS-26 | *NCOR1* |
| RP11-46M22 | 17P11.12 | Chr17:16963277-17125414 | 1 | KMS-26 | *D17S1280* |
| RP11-758H9 | 17q23.2 | Chr17:55129450-55139638 | 1 | AMO1 | *CG1-147* |
| BAC-BCL2 | 18q21.33 | Chr18:68941558-59136910 | 1 | KMS-12PE | *BCL2* |
| RP11-28F1 | 18q21.33 | Chr18:59058634-69216681 | 1 | KMS-12PE | *FVT1* |
| **Homozygous deletion** | | | | | |
| RP11-220M1 | 1p32.3 | Chr1:51531955-51597016 | 1 | KM-5 | *EPS15* |
| RP11-70L8 | 8p21.3 | Chr9:21732608-21901258 | 1 | KMS-5 | *MTAP* |
| RP11-145E5 | 9p21.3 | Chr8:21957751-21985097 | 1 | KMS-5 | *CDKN24* |
| RP11-681O17 | 11q22.1 | Chr11:100414312-100506465 | 3 | KMS-20. KMS-28BM. KMS-28PE | *PGR* |
| RP11-111A13 | 11q22.1 | Chr11:101517505-101688954 | 4 | KMS-20, KMS-28BM, KMS-28PE, MOLP-6 | *YAP1* |
| RP11-864G5 | 11q22.1 | Chr11:101638105-101818088 | 3 | KMS-20. KMS-28BM, KMS-28PE | *ClAP1* |
| RP11-31EO6 | 11q22.2 | Chr11:101724636-101939137 | 2 | KMS-20. KMS-28PE | *MMP7* |
| RP11-760P6 | 11q22.2 | Chr11:102010610-102191046 | 2 | KMS-20, KMS-28PE | *MMP1* |
| RP11-2122 | 11q22.3 | Chr11:102613896-102771594 | 2 | KMS-28BM, KMS-28PE | *DYNEIN* |
| RP11-264E20 | 11q24.3 | Chr11:127930597-128090778 | 1 | KMS-28PE | *ETS1* |
| RP11-744N12 | 11q24.3 | Chr11:128069198-128187520 | 1 | KMS-28PE | *FU1* |
| RP11-417P24 | 14q32.33 | Chr14:105278722-105753232 | 6 | KM-5, KM-7,ILKM-10. KMS-18.OPM-2 | *IGHG1* |

| | | | | | |
|---|---|---|---|---|---|
| ^{a}Based on UCSC Genome Browser(May 2004 Assembly) ^{b}Representative candidate tumor-related genes or markers located around BAC | | | | | |

Genes exhibiting homologous deletion were observed in 11 out of 28 cell lines, and deletion of MTAP and CDKN2A/p16 in the 9p21.3 region was observed in KMS-5 cell. However, high-level amplification was observed in 11 out of 28 cell lines, and 16 genes were identified. Among them, the POU2AF1 and PPP2R1B genes in 11q23 region were found to be amplified in AMO1 and MOLP-2 cells. Since gene amplification has great pathological and clinical significance concerning tumors, and elucidation of such gene amplification influences development of therapeutic methods, this 11q23 region was analyzed intensively.

### Example 2: FISH analysis of AMO 1 and MOLP-2 cells

The chromosome 11q23 region observed by the array CGH technique in Example 1 was subjected to FISH analysis in accordance with a conventional technique using BAC clones of RP11-792P2 (Inoue J, Otsuki T, Hirasawa A, et al., Am J Pathol.; 165: 71-81, 2004) (Fig. 2). In addition to the FISH signals (arrows) at the original chromosome positions, strong amplification signals (wedge-shaped) comprising multiple copies of genes were observed in both cell lines. This indicates that gene amplification actually took place.

### Example 3: Narrowing-down of amplified region by FISH analysis in AMO1 and MOLP-2 cells, and expression analysis of constitutive gene

FISH analysis was carried out using 15 types of HAC clones substantially typified by RP11-792P2. Also, the copy number of the corresponding genomic DNA region was schematically expressed based on fluorescent signal intensities (Fig. 3, left). The vertical axis represents the BAC clone IDs in the 11q23 region and relative positions thereof The homogeneously stained regions with the highest luminescence (i.e., the homogenously staining regions (HSRs)) were substantially the same between both cells, and the regions of interest were each consequently determined to be an about 3-Mb (megabase) region between RP11-25I9 and 708L7.

Subsequently, the AMO1, RDX, ARHGAP20, POU2AF1, SNF1LK2, PPP2R1B, and ALG9 genes located in the determined 11q23 region were subjected to PCR for the purpose of simple quantification. As a control example for the RT-PCR expression level, GAPDH, the expression level of which was known to be less likely to be influenced by cell species and conditions, was used. Total RNA was recovered from cells at the logarithmic growth phase, and cDNA was prepared in accordance with a conventional technique. Primers and conditions specific to each gene were determined in advance, followed by PCR and electrophoresis in 3% agarose gel. The gel image was obtained using an LAS-3000 (Fuji Photo Film Co., Ltd.) and the obtained image was analyzed using Multi Gauge software (Fuji Photo Film Co., Ltd.) (Fig. 3, right). In addition to the AMO1 and MOLP-2 cell lines, the 11q23 regions of which had been amplified, KMM1 and KM-5, which did not exhibit amplification in this region were used. Further, LCL (lymphocyte clones) obtained from a healthy volunteer was used as control. Table 2 shows the summary of the results of quantification.

**Table 2: Comparison of expression analysis of genes in the 11 q23 region in the cells wherein the genome DNA of the 11 q23 region was amplified and in the cells wherein the genome DNA of the 11q23 region was not amplified**

| **Known gene** | **Amplified cells** | | **Non-amplified cells** | | **Fold increase (/reference value)^{a}** | |
|---|---|---|---|---|---|---|
| | **AMO1** | **MOLP-2** | **KMM1** | **KM-5** | **AMO1** | **MOLP-2** |
| RDX | 0.89 | 0.79 | 0.88 | 1.33 | | |
| FDX1 | 1.22 | 1.36 | 0.79 | 1.23 | | |
| ARHGAP20 | 0.48 | 1.71 | 1.39 | 2.93 | | |
| POU2AF1 | 0.54 | 0.60 | 0.06 | 0.18 | **4.50** | **5.00** |
| BTG4 | N.T.^{b} | N.T.^{b} | N.T.^{b} | N.T.^{b} | | |
| LAYN | N.T.^{b} | N.T.^{b} | 0.55 | N.T.^{b} | | |
| SNF1LK2 | 0.75 | 0.32 | 0.60 | 0.51 | | |
| PPP2R1B | 0.71 | 0.93 | 1.05 | 0.17 | | |
| ALG9 | 0.57 | 0.44 | 0.26 | 0.33 | 1.90 | 1.50 |

| | | | | | | |
|---|---|---|---|---|---|---|
| ^{a} The expression level of each gene in each cell line was divided by the average value of that in KMM1 and KM-5 cell lines (reference value), which have normal copy-numbers of 11q, after normalization with GAPDH, and recorded as a fold increase in relative expression level. Fold increases in relative expression levels >2.0 were considered significant and shown in bold type. The single gene that was significantly overexpressed as a consequence of amplification of 11q23 is POU2AF1. ^{b} N.T. indicates that expression level is below the lower limit of quantification. | | | | | | |

In the regions that have been narrowed down in AMO1 and MOLP-2 cells, the expression level of the POU2AF1 gene was found to be elevated to a level 4.5 to 5.0 times higher than the average expression level in the cells (KMM1, KM-5) in which gene amplification was not observed.

### Example 4: Confirmation of protein expression level of POU2AF1 gene in multiple myeloma cell line

Concerning 8 types of multiple myeloma cell lines, the expression levels of POU2AF1 proteins were quantified by Western blotting (Fig. 4). The cells were lysed in RIPA buffer (10 mM Tris-HCl, 150 mM NaCl, 1 mM EDTA, 1% sodium deoxycholate, 0.1% SDS, and 1% Triton X-100, pH 7.4) containing protease-inhibitor cocktails (Roche Diagnostics), protein concentrations were measured via BCA assay (Pierce Chemical), and 10 µg each thereof was electrophoresed on SDS-polyacrylamide gel. The resultants were transferred onto difluoride membranes, the primary detection was carried out using an auti-POU2AF1 antibody (Santa Cruz Biotechnology) and an anti-β-actin antibody (Sigma) as a control, and detection was then carried out by developing color with the use of a peroxidase-bound secondary antibody using an enhanced electrochemiluminescence system (Amersham). In the figure, 2 types of bands are observed in the POU2AF1 protein, and p35 was detected in addition to p34 of small molecular weight. p34 is an isoform resulting from post-transcriptional modification of p35, and its satisfactory effects of enhancing transcription activation for the OCT binding factor 1 have been reported (Yu X. et al., Immunity 14: 157-167, 2001). The lower part of the figure shows the copy number of the POU2AF1 gene in genomic DNA determined via FISH. As expected, the AMO1 and the MOLP-2 cells in which amplification of the POU2AF1 genes were observed in their genomic DNAs exhibited higher expression levels of POU2AF1 proteins than other cells.

### Example 5: Effects of inhibiting expression of POU2AF1 protein and cell growth with the addition of siRNA of the POU2AF 1 gene to the cell

siRNAs of POU2AF1 were designed as POU2AF1-A: CACCUUACACCGAGUAUGU (SEQ ID NO: 3) and POU2AF1-B:GGUUCUGUGUCUGCAGU (SEQ ID NO: 4), and were purchased (Japan Bioservice). In 2 x 10⁶ of AMO1 and the KMS-21BM cells in which expression of POU2AF1 gene was observed, 200 nM of each primer was transduced with the aid of Nucleofector (Germany), and the efficiency thereof was analyzed by Western blotting by monitoring via fluorescence-labeled pmaxGFP analysis in the same manner as in Example 4 (Fig. 5A). An anti-β-actin antibody was used as a control. As expected, inhibition of POU2AF1 protein expression was observed with the addition of siRNA. Also, siRNA of POU2AF1 was transduced into 1 x 10⁴ AMO1 and KMS-21BM cells, the resultants were sowed on a 96-well plate, and the viable cell count was determined by water-soluble tetrazolium salt (WST) assay (Cell counting kit-8; Dojindo) with the elapse of time (Figs. 5B and 5C). The experiment was carried out twice in triplicate, and an asterisk is used for results that were determined to be significant (p < 0.05) via the unpaired Student's t test. In both cells, cell growth was inhibited by introducing siRNA of POU2AF1.

### Example 6: Establishment of POU2AF1 gene-expressing cell line in KMS-11 cells, distribution thereof, and observation of influence on viable cell count

An expression vector (pCMV-Tag3-p34-POU2AF1) was constructed so as to express the full-length POU2AF1 gene (p34) wherein Myc was bound to the N-terminus. The resulting vector was introduced into the KMS-11 cell in which the expression level of endogenous POU2AF 1 gene was low using Nucleofector, and drug selection was carried out for 3 weeks with G418 (50 µg/ml). The occurrence of POU2AF1 gene expression was detected using a solution of disrupted cells via Western blotting using an anti-Myc antibody (Fig. 6A). Simultaneously, pCMV-3B that did not contain any foreign gene was introduced into the KMS-11 cell, and the resultant was designated as a "mock control." As expected, the recombinant POU2AF1 proteins were detected in cells into which the gene had been introduced. The transformed cells were cultured on a glass slide with a Shandon cytospin (Thermo), centrifuged, immobilized with 10% formamide and stained with an anti-Myc polyclonal antibody (Cell Signaling Technology) overnight. After the reaction, the antibody was stained with the Alexa-488-labeled sheep-derived anti-rabbit antibody (Molecular Probes), and the stained antibody was observed under a fluorescence microscope. Simultaneously, nuclear staining of DAPI was carried out. The hues were adjusted and represented as "merge" (Fig. 6B). POU2AF1 was found to be an intranuclear protein, it was found to be nuclearly stained as with the case of DAPI, and it was confirmed that POU2AF1 exhibited its inherent distribution patterns. The mock control and POU2AF1 gene-expressing cells were subjected to WST assay by determining the cell growth rate as the viable cell count (Fig. 6C). The cell growth rate was significantly increased by POU2AF1 gene introduction. Thus, the remarkable effects of such gene for cell growth acceleration were verified.

### Example 7: TNFRSF17 as a POU2AF1 gene-regulating gene

Based on the experiment using a POU2AF1 gene-knockout mouse, involvement of the POU2AF1 gene in expression of genes other than immunoglobulin genes has been reported (Teitell MA. et al., Trends Immunol. 24: 546-553, 2003). As genes associated with development or advancement of cancer, cell growth, cell adhesion, or migration, BAFFR, TNFRSF17 (tumor necrosis factor receptor superfamily, member 17), Bcl-2, cyclin D3, osteopontin, and the like are known. As a result of real-time RT-PCR analysis, however, TNFRSF17 was found to be the only gene that would exhibit the same behavior as the POU2AF1 gene in the multiple myeloma cells (data is not shown). Thus, gene expression behavior of TNFRSF 17 was thoroughly analyzed (Fig. 7). At the outset, 200 nM each of siRNA of POU2AF1-A and POU2AF1-B were transduced into 2 x 10⁶ AMO1 and KMS-21BM cells (only POU2AF1-B regarding the KMS-21BM cells) using Nucleofector, and expression of the TNFRSF17 gene was analyzed by real-time RT-PCR 48 hours thereafter (Fig. 7A). In both cells, the expression levels of the TNFRSF17 genes were found to become lower with the knocking down of the POU2AF1 gene.

In Example 6, the KMS-11 cell line expressing the POU2AF1 gene was prepared with the mock control. The TNFRSF17 gene expression behaviors in those cells were compared by real-time RT-PCR (Fig. 7B). Thus, the expression level of the TNFRSF17 gene was found to increase in a cell in which the POU2AF1 gene was forced to express. A transcription-factor-binding site in the 5' region of the human TNFRSF17 gene in genomic DNA was searched by using the database (RecGroupScan; http://wwwmgs.bionet.nsc.ru/mgs/programs/yura/RecGropScanStart.html). As a result, the presence of the octamer site, the transcription of which is promoted by POU2AF1, was observed at a site 3,642 bases upstream of the initiation point of transcription (+1) (Fig. 7C). Regarding chromatins of the AMO1 cell and the KMS-11 cell (mock control, POU2AF1 gene-expressing cell), whether or not an immune precipitate using an antibody against the POU2AF1 protein had the octamer site was then inspected by PCR (Fig. 7D). As indicated by an arrow in Fig. 7C, PCR primers that sandwich the octamer site were used. The cells were subjected to immunoprecipitation using an anti-POU2AF1 antibody (Santa Cruz Biotechnology), and 1/100 of the resultant was used as a template for PCR. The "input" indicates a chromatin material before immunoprecipitation, and (-) indicates a negative control without any immunoprecipitation antibody. As indicated by an arrow, the octamer site bound to the POU2AF1 protein was detected in the POU2AF1 gene-expressing cell and in the AMO1 cell that would express the endogenous POU2AF1 gene.

### Example 8: Promoter-reporter assay in combination with the POU2AF1 expression vector using an octamer-site containing region upstream of the TNFRSF17 gene

A 307-bp DNA region containing the octamer site upstream of the TNFRSF17 gene was cloned by PCR, a region containing an octamer site of a wild type (wt; TTTAGCAT) and a region containing an octamer site of a mutant (Mut; TTCCGCAT) were introduced into pGL3-promoter vectors (Promega), and the resultants were designated as a pGL3-TNFRSF17-wt octamer and a pGL3-TNFRSF17-Mut octamer, respectively. The two types of luelforuse-promoter-reporter vectors were transduced into the KMS-11 cell together with p34 or p35 of pCMV-Tag3-POU2AF1 and internal reference vectors, phRL-TK (Promega), for correcting transduction efficiency using the lipofectamine 2000 (Invitrogen). Luminescence was assayed using the dual-luciferase reporter assay system (Promega) 48 hours thereafter. The activity of the cell into which pGL3-TNFRSF17-wt octamer and pCMV-Tag3 (mock) had been transduced was designated as 1, and relative luciferase activity was shown (Fig. 8). The POU2AF1 isoforms, p35 and p34, both exhibited higher reporter activity than the control having an octameric site of the wild type. Activity, however, was lowered by variation in the binding site. This indicates that the POU2AF1 binds to a transcriptional factor, OCT1 or OCT2, and enhances its activity. Thus, the POU2AF1 gene was found to regulate expression of the TNFRSF17 gene by enhancing its activity.

### Example 9: Correlation of mRNA expression of POU2AF1 gene and TNFRSF17 gene

The POU2AF1 and TNFRSF17 gene expression levels in the multiple myeloma cell lines and the primary cells established from the multiple myeloma clinical sample were measured by quantitative PCR, and the determined values were represented in terms of the ratio relative to β-actin and β2-microglobulin (β2M) (Fig. 9A: cell line; B: primary cell). Expression of the POU2AF1 gene was highly correlated with that of the TNFRSF17 gene both in the cell lines and in the primary cells, and both values were significant p values (significance determined by an unpaired Student's t test of p < 0.05). Thus, TNFRSF17 gene expression was found to be positively correlated with POU2AF1 gene expression not only in the cell line but also in a clinical sample.

### Example 10: Effects of inhibiting cell growth by inhibiting TNFRSF17 gene expression using siRNA

The AMO1 cells in which the expression level of the POU2AF1 gene was high and the KMS-21BM cells in which expression of the POU2AF1 gene was observed were used. A nonspecific sequence was employed as control siRNA, and siRNA of the FOU2AF1 gene and the TNFRSF17 gene (200 nM each, M-011217-00, Dharmacon, Inc.) were transduced. POU2AF1 and TNFRSF17 gene expression was assayed by quantitative PCR using β-actin as an indicator 48 hours thereafter (Fig. 10A: POU2AF1; Fig. 10 B: TNFRSF17). As expected, POU2AF1 gene expression was inhibited by inhibiting the POU2AF1 gene by siRNA; however, POU2AF1 gene expression was not inhibited by siRNA of the TNFRSF17 gene, and TNFRSF17 gene expression was inhibited by inhibiting the POU2AF 1 gene by siRNA. This indicates that the POU2AF1 gene inhibits TNFRSF17 gene expression at a site upstream of the TNFRSF17 gene. The viable cell counts in the AMO1 and the KMS-21BM cells after the inhibition of the TNFRSF17 genes by siRNA were determined by the WST technique (Fig. 10C). In both cells, cell growth began to be significantly inhibited 4 days after the introduction of siRNA of the TNFRSF17 gene (an asterisk represents the unpaired Student's t test (p < 0.05)). As is apparent from these results, TNFRSF17 gene expression is positively regulated by POU2AF1 gene expression, and the effects thereof are exhibited as the cell growth effects.

### (Conclusion of Example)

(1) Abnormality in the POU2AF1 gene was observed via array CGH screening in combination with expression analysis of amplified genes of DNAs in 28 types of multiple-myeloma-derived cells.
(2) POU2AF1 gene expression was found to be located upstream of TNFRSF17 gene expression, and to play its function by positively regulating TNFRSF17 gene expression, consequently positively regulating cell growth, and accelerating cell growth.

### Effects of the Invention

The present invention enables typing of tumor and accurate understanding of an indication of tumorigenesis and the extent of recovery from tumor of a multiple myeloma cell specimen. Also, inhibition of the POU2AF1 gene transcript amplified in the multiple myeloma cell enables inhibition of growth of multiple myeloma cells. Specifically, the present invention provides an inhibitor for transcription of the POU2AF1 gene, the tumorigenic functions of which have been newly found, or an antitumorigenic agent comprising a loss-of-function phenotype of the POU2AF1 protein encoded by such gene. Such agents are very useful from the clinical point of view in terms of improvement in treatment or prognosis of tumors based on tumor individuality, and also from the viewpoint of basic research concerning tumors. Further, assay of the expression level of messenger RNA of the POU2AF1 gene, determination of the amount of such gene in genomic DNA, or assay of the POU2AF1 protein level enables genetic typing of tumor of a patient with multiple myeloma.

## Claims

1. A method for detecting cancer which comprises detecting and/or typing tumorigenesis of a specimen by employing gene amplification in the chromosome 11 q23 region of the specimen as an indicator.

2. The method for detecting cancer according to claim 1 wherein the gene is any of the RDX, FDX1, ARHGAP20, POU2AF1, LAYN, SNF1LK2, PPP2R1B, or ALG9 genes.

3. The method for detecting cancer according to claim 1 wherein an indicator for amplification is at least 1.32 times higher than that of a normal specimen.

4. The method for detecting cancer according to claim 1 wherein the specimen is of multiple myeloma.

5. The method for detecting cancer according to claim 1 wherein the cancer is multiple myeloma.

6. A method for detecting multiple myeloma which comprises detecting and/or typing the multiple myeloma tumorigenesis of a specimen by employing amplification of the POU2AF1 gene as an indicator.

7. The method for detecting cancer according to claim 1 wherein gene amplification is detected via a DNA chip technique, Southern blotting, Northern blotting, PCR, real-time RT-PCR, FISH method, CGH method, gene amplification, RFLP detection, nucleotide sequencing, or the array CGH method.

8. A method for detecting cancer which comprises detecting and/or typing tumorigenesis of a specimen using, as an indicator, the elevated expression level of any of the RDX gene, the FDX1 gene, the ARHGAP20 gene, the POU2AF1 gene, the LAYN gene, the SNF1LK2 gene, the PPP2R1B gene, or the ALG9 gene of the specimen.

9. A method for inhibiting cell growth which comprises introducing *in vitro* siRNA, shRNA, an antisense oligonucleotide, or a loss-of-function gene of a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene into a tumor cell.

10. A method for inhibiting cell growth which comprises introducing *in vitro* a loss-of-function protein of a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene into a tumor cell.

11. A cell growth inhibitor which comprises siRNA, shRNA, antisense oligonucleotide, or a loss-of-function gene of a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene.

12. A cell growth inhibitor which comprises a loss-of-function protein of a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene.

13. A method for activating cell growth which comprises introducing *in vitro* a gene selected from among the RDX gene, the FDX1 gene, the ARHGAP20 gene, the PDU2AF1 gene, the TNFRSF17 gene, the LAYN gene, the SNF1LK2 gene, the PPP2R1B gene, and the ALG9 gene into a cell.

14. A cell growth activator which comprises a gene selected from among the RDX gene, the FDX1 gene, the ARHGAP20 gene, the POU2AF1 gene, the TNFRSF17 gene, the LAYN gene, the SNF1LK2 gene, the PPP2R1B gene, and the ALG9 gene.
